# EUROPEAN PATENT SPECIFICATION

(11) **EP 0 489 728 B1**
(45) Date of publication and mention of the grant of the patent: **29.01.1997**
(21) Application number: 89910649.6
(22) Date of filing: 16.08.1989
(51) Int. Cl.: A61L 27/00

(54) **CHEMICAL COMPOUNDS**
CHEMISCHE VERBINDUNGEN
COMPOSES CHIMIQUES

(30) Priority: 19.08.1988 GB 8819755
(43) Date of publication of application: 17.06.1992
(73) Proprietor: ED. GEISTLICH SÖHNE AG FÜR CHEMISCHE INDUSTRIE, CH-6110 Wolhusen Lucerne (CH)
(72) Inventor: PFIRRMANN, Rolf, Wilhelm, CH-6006 Lucerne (CH); Geistlich, Peter, 6362 Stansstad (CH)
(74) Representative: Holmes, Michael John
(86) International application number: GB8901020
(87) International publication number: WO9001955

(56) References cited:
- EP-A- 0 141 004
- EP-A- 0 182 483
- EP-A- 0 243 178
- WO-A-86/07265
- US-A- 2 968 593

## Description

This invention relates to improvements in bone mineral products of large specific surface area.

Bones from slaughtered animals are an inexpensive raw material available in large quantities. They contain 50 to 60% of very finely crystallized hydroxylapatite bonded by collagenic tissue and containing significant qualities of proteinaceous and other matter as well as associated fat and muscle tissues. In view of its biologically formed crystal structure it can also be considered as a highly biocompatible prosthetic bone replacement. Owing to its large specific surface it can also be used, for example, as an adsorbent or as a support for slow release medication.

Natural bone mineral comprises hydroxyapatite-like crystallites with a particular degree of crystallinity, habit and size (irregular plate-like morphology, 5-10nm in thickness 10-50 nm in length) and surface chemistry resulting from the calcium to phosphate ratio (37.5-38.0% calcium and 15.5-5-19.0% phosphorus). Also present in the natural bone mineral are small amounts of noncrystalline entities and other calcium phosphate crystalline phase including the minerals Brushite and Whitlockite, and octa-calcium phosphate. The inorganic phase of bone contains porosity including ultrastructural interstices (10-100 nm) between the crystallities occuring naturally and produced by removal of the organic phase, and microscopic spaces (1-20 microns) including osteocyte lacunae, canaliculi, vascular channels, volkman's canals, and the canals of haversian systems (109-500 nm). The specific surface area, which is a measure of porosity is in the range 50 to 100 m²/gm as determined by mercury porosimetry. The crystallinity of bone mineral can be characterized by X-ray diffraction and the porosity and crystallite morphology and size by electron microscopy. Small amounts of nonapatitic crystallites can be detected by thermogravimetric analysis.

We have found however, that the composition and structure of natural bone mineral cannot be duplicated by products formed in vitro or by naturally occurring hydroxyapatites prepared previously. Two methods for the purification of natural bone mineral have been proposed namely calcination and solvent extraction.

The temperature needed during calcination for the incineration of the organic constituents of the bones are rather high. This leads to extensive recrystallization of the mineral part with formation of much coarser crystals. The so formed material exhibits a relatively small specific surface. Thus, such material is not readily remodelled to form new bone on implantation and implants may remain unremodelled indefinitely although this may be acceptable for some purposes.

In the extraction processes the proteins are extracted from degreased bone with a suitable solvent. The resulting bone mineral is then washed to remove the solvent.

In both cases, when organic impurities are removed from the natural bone to leave only the bone mineral, the strength of the material is greatly reduced and the individual pieces of purified bone mineral are consequently extremely brittle. This renders handling of the material difficult and may lead to undesirable effects on implantation. We have found, however, that adequate strength can be imparted to the brittle bone mineral by providing, at least on the surface, collagen which is non-antigenic and which is resorbable on implantation.

US-A-4 314 380 describes purified animal bone being impregnated and coated with a solution of atelocollagen.

According to the present invention we provide a purified particulate bone mineral product having a porous structure derived from natural bone for use in medicine, the particles of said mineral being substantially free from all endogenous organic material and having at least at the surface thereof resorbable, physiologically compatible, macromolecular material comprising a felt of water-insoluble, collagen fibres, optionally together with gelatin whereby the strength of the bone mineral particles is increased solely by said macromolecular material.

The macromolecular material may be used to provide a matrix for the particulate bone mineral from which shaped articles may be formed. In this case, it is particularly preferred to use a fibrous macromolecular substance such as a fibrous collagen of

Type I or Type I-III, together with a gel forming macromolecular substance such as gelatin. The weight ratio of the fibrous material to the bone mineral may, for example, be in the range 1:20 to 3:20 e.g. about 1:10. The gel phase advantageously amounts to 2 to 8% by weight of the bone mineral, e.g. about 5%. Where gelatin is used as the gel phase, it may be lightly cross-linked, e.g. with about 0.2% formaldehyde.

We have found that bone mineral treated in accordance with the invention is far less subject to the problem of particle migration than uncoated bone mineral particles.

The particulate bone mineral product according to the invention may be used as a remodelling implant or prosthetic bone replacement, for example in orthopoedic surgery, including hip revisions, replacement of bone loss, e.g. in traumatology, remodelling in maxillo-facial surgery or filling periodontal defects and tooth extraction sockets, including ridge augmentation. The impregnated particulate material of the invention may thus be used for packing into a variety of bone cavities and its reduced brittleness is significant in aiding the handling and packing procedure.

The fibre matrix embodiment of the invention lends itself to the preparation of moulded shapes for bone remodelling. The dry matrix material, usually in the form of sheets, may be cut to approximate size, moistened to soften the matrix material and moulded the desired shape.

The purified bone mineral may, for example, be a product as described in International Patent Application WO 86/07265 (PCT/GB86/00310). Such products may be prepared by rigorously de-greasing particulate bone, e.g. bovine femurs, and treating with ammonia or an organic amine to degrade residual protein followed by extensive water washing. Such material remains resorbable on implementation, assisting the remodelling process.

It has also be proposed to prepare purified bone mineral by calcinating particulate cancellous or cortical bone e.g. at 900°C for 24 hours. Such calcined bone mineral is of use where permanent, non-resorbable implants are required, for example in ridge augmentation. In either way after removal of organic material, the bone is excessively brittle and its strength is greatly improved by treatment according to the invention.

Where the bone is to be used as a drug carrier, as indicated in the above International Patent Application the bone mineral may usefully carry one or more absorbed drugs or other physiologically active substances.

Physiologically active substances which may be adsorbed onto the bone mineral are preferably at least partially water-soluble and include anti-bacterial substances such as antibiotics, e.g. penicillins, cephalosporins, aminoglycosides etc., sulphonamides and, in particular, condensation products of formaldehyde with taurinamide or N-substituted taurinamide. The latter compounds may be represented by the formula 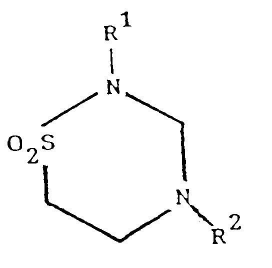 where R¹ is hydrogen or a C₁₋₄ alkyl group and R² is hydrogen or a group of the formula 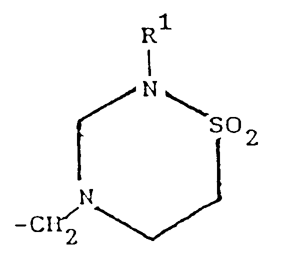 wherein R¹ has the above meaning.

The compound of formula (I) in which R¹ and R² are both hydrogen is taurultam while the compound in which R¹ is hydrogen and R² has the formula (II) is taurolidine. These compounds act as methylol transfer agents and are effective not only in destroying both gram negative and gram positive bacteria but also in inactivating both endotoxins and exotoxins produced by the bacteria.

Other useful physiologically active substances include proteins and polypeptides capable of assisting bone regeneration especially non-collagenous proteins derived from bone matrix and bone cells. These include mitogenic factors such as skeletal growth factor and morphogenic and angiogenic factors as well as transforming bone growth factor. Growth factors from the natural bone matrix such as ossein or more preferably osteopoietin are particularly beneficial.

It will be appreciated that physiologically active substances may alternatively or additionally be incorporated in the macromolecular substance e.g. impregnated gelatin. This is particularly suitable for proteins such as the bone growth factors set out above.

The bone mineral will normally be in the form of particles of average diameter in the range 0.1 to 10mm. Pieces of cancellous bone will commonly be in the upper size range, e.g. 2 to 10mm in diameter, and may provide shaped inserts; such cancellous bone is particularly brittle due to the large size of the pores therein and benefits very greatly from treatment according to the invention. Particles for incorporation into collagen fibre will commonly be of cortical bone and will generally be in the size range 0.1 to 3mm, preferably 0.25 to 2mm. It may be beneficial to the close packing of the bone mineral particles to use a mixture of two or more particle sizes, e.g. 0.25 to 1mm and 1 to 2mm or a broad range e.g. 0.25 to 2 mm.

The purified bone mineral may be obtained, for example, by the method described in the above International Patent Application. Thus, for example, fats may be removed using one or more conventional fat solvents such as ethers, e.g. diethyl ether; ketones e.g. acetone; or hydrocarbons or halogenated hydrocarbons e.g. heptane or methylcylcohexane or toluene.

It may be advantageous to remove an extractant such as toluene by an intermediate extraction with a water miscible solvent such as ethanol before proceeding further. Collagen material may be dissolved using proteolytic agents such as bases e.g. potassium hydroxide in glycerol, or organic bases such as amines, e.g. ethylene diamine, or amides such as formamide, preferably at elevated temperatures. Such agents are preferably water-miscible to facilitate removal by water washing. Especially good results have been obtained using bone extracted with refluxing ethylene diamine.

Extraction may advantageously be continued at each stage, if necessary with changes of solvent, until no further material is extracted, e.g. for periods up to one or two weeks. It may be advantageous to comminute further after initial protein removal since the bone is more readily fractured at that stage than before extraction. After treatment with base, excess solvents are rigorously removed e.g. by evaporation and/or, where suitable, water washing. Water washing may for example be effected for long periods at 100°C to ensure that all the solvent and dissolved substances are removed.

The material is normally subjected to a drying step. It may be convenient to sterilise the material at this stage, e.g. by heat treatment which may effect further purification. Absorption and/or adsorption of the physiologically active substance is preferably effected by immersing the treated bone mineral in an aqueous solution thereof preferably under sterile conditions. The concentration of the active substance is preferably relatively high to facilitate adsorption and/or absorption and will depend in part on the solubility of the active material.

The following Examples are given by way of illustration only:

### Preparation

Bovine femur bones were boiled in hot water until clean, comminuted to a particle size of 5 to 10mm. and extracted under reflux with toluene for 24 hours in a Sohxlet apparatus. The material was further extracted with ethanol to remove toluene and then extracted at elevated temperature with an azeotropic mixture of ethylene diamine and water (85:15) for 8 days, with several changes of solvent until substantially no further organic material was extracted. After extraction the material was washed with hot water at 100°C for 10-14 days with frequent changes of water. The product was then air dried at 100°C.

The dried product was further comminuted to an average particle size of 0.2 to 2 mm and sterilized in the autoclave.

Pieces of bovine femur spongeosa bone, typical diameter 10mm, were purified by the same technique, omitting the final granulation.

### Example 1

2.0g of collagen fibre felt were comminuted with 500g distilled water in a blender. This dispersion was centrifuged and the supernatant water removed. To the resulting collagen fibre slurry were added 17.5g of granulated cortical bovine bone purified by the above procedure, followed by thorough mixing and removal of water by suction (70mm). The granulated bone had a particle size 0.5 to 1.0 mm. After removal of water 5 mls of a 9% w/w aqueous gelatin solution were added (cross-linked with 0.6% of 35% aqueous formaldehyde) and the mixture again suction dried.

The sponge mass was cut into pieces and dried in vacuo at 60°C. The pieces of sponge were packed into polyethylene containers and sterilised by gamma irradiation.

## Claims

1. A purified particulate bone mineral product having a porous structure derived from natural bone for use in medicine, the particles of said mineral being substantially free from all endogenous organic material and having at least at the surface thereof resorbable, physiologically compatible, macromolecular material comprising a felt of water-insoluble, collagen fibres, whereby the strength of the bone mineral particles is increased solely by said macromolecular material.

2. A product as claimed in claim 1 in which the collagen fibres provide a matrix for the particulate bone mineral.

3. A product as claimed in claim 1 or claim 2 including a gel-forming macromolecular substance.

4. A product as claimed in claim 3 in which the gel-forming substance is gelatin.

5. A product as claimed in any of claims 1 to 4 in which the weight ratio of the collagen fibres to the bone mineral is in the range 1:20 to 3:20.

6. A product as claimed in claim 3 in which the gel phase amounts to 2 to 8% by weight of the bone mineral.

7. A product as claimed in any of the preceding claims in which the purified bone mineral is prepared by rigorously de-greasing particulate bone and treating it with ammonia or an organic amine to degrade residual protein followed by extensive water washing.

8. A product as claimed in any of the preceding claims in which the bone mineral carries one or more absorbed drugs or other physiologically active substances.

9. A product as claimed in claim 8 in which the bone mineral carries taurolidine and/or taurultam.

10. A product as claimed in claim 8 in which the bone mineral or collagen fibres carries one or more proteins or polypeptides capable of assisting bone regeneration.

11. A product as claimed in claim 10 in which the proteins or polypeptides include mitogenic, morphogenic or angiogenic growth factors, transforming bone growth factor, ossein or osteopoietin.

12. A product as claimed in any of the preceding claims which is in the form of particles of average diameter in the range 0.1 to 10 mm.

## Patentansprüche

1. Reines, partikelförmiges Knochenmineral-Produkt, mit poröser Struktur, abgeleitet von natürlichen Knochen zur Verwendung in der Medizin, wobei die Partikel des Minerals von jedwedem endogenen organischen Material im wesentlichen frei sind und wenigstens an der Oberfläche resorbierbares, physiologisch kompatibles makromolekulares Material aufweisen, das ein Geflecht wasserunlöslicher Kollagenfasern umfaßt, wobei die Festigkeit der Knochenmineral-Partikel ausschließlich durch das makromolekulare Material vergrößert wird.

2. Produkt nach Anspruch 1, in dem die Kollagenfasern eine Matrix für das partikelförmige Knochenmineral bilden.

3. Produkt nach Anspruch 1 oder 2, das eine gelbildende, makromolekulare Substanz umfasst.

4. Produkt nach Anspruch 3, wobei die gelbildende Substanz Gelatine ist.

5. Podukt nach einem der Ansprüche 1 bis 4, wobei das Gewichtsverhältnis der Kollagenfasern zum Knochenmineral im Bereich von 1:20 bis 3:20 liegt.

6. Produkt nach Anspruch 3, wobei die Gelphase 2 bis 8 Gew.-% des Knochenminerals ausmacht.

7. Produkt nach einem der vorhergehenden Ansprüche, wobei das reine Knochenmineral hergestellt wird, indem man partikelförmigen Knochen gründlich entfettet und ihn mit Ammoniak oder einem organischen Amin behandelt, um Proteinreste abzubauen und anschließend extensiv mit Wasser wäscht.

8. Produkt nach einem der vorhergehenden Ansprüche, wobei das Knochenmineral ein oder mehrere absorbierte Medikamente oder andere physiologisch aktive Substanzen trägt.

9. Produkt nach Anspruch 8, wobei das Knochenmineral Taurolidin und/oder Taurultam trägt.

10. Produkt nach Anspruch 8, wobei das Knochenmineral oder die Kollagenfasern ein oder mehrere Proteine oder Polypeptide trägt, die befähigt sind, Knochenregeneration zu unterstützen.

11. Produkt nach Anspruch 10, wobei die Proteine oder Polypeptide mitogene, morphogene oder angiogene Wachstumsfaktoren, Transforming Bone Growth Factor, Ossein oder Osteopoietin umfassen.

12. Produkt nach einem der vorhergehenden Ansprüche, das in Form von Partikeln mit einem durchschnittlichen Durchmesser im Bereich von 0,1 bis 10 mm vorliegt.

## Revendications

1. Produit minéral osseux, particulaire, purifié, ayant une structure poreuse, dérivé d'os naturels, à utiliser en médecine, les particules dudit minéral étant essentiellement dépourvues de toutes les matières organiques endogènes et ayant, au moins en surface, une matière macromoléculaire, physiologiquement compatible, résorbable, comprenant un feutre de fibres de collagène insolubles dans l'eau, de sorte que la résistance mécanique des particules minérales osseuses est augmentée uniquement par ladite matière macromoléculaire.

2. Produit selon la revendication 1, dans lequel les fibres de collagène constituent une matrice pour le minéral osseux particulaire.

3. Produit selon la revendication 1 ou la revendication 2, comprenant une substance macromoléculaire formant un gel.

4. Produit selon la revendication 3, dans lequel la substance formant un gel est la gélatine.

5. Produit selon l'une quelconque des revendications 1 à 4, dans lequel le rapport pondéral des fibres de collagène au minéral osseux est dans la gamme de 1:20 à 3:20.

6. Produit selon la revendication 3, dans lequel la phase gel constitue 2 à 8% en poids du minéral osseux.

7. Produit selon l'une quelconque des revendications précédentes, dans lequel le minéral osseux purifié est préparé par un dégraissage rigoureux d'un os particulaire et par traitement de ce dernier avec de l'ammoniac ou une amine organique pour dégrader les protéines résiduelles, suivi d'un lavage complet à l'eau.

8. Produit selon l'une quelconque des revendications précédentes, dans lequel le minéral osseux contient un ou plusieurs médicaments ou d'autres substances physiologiquement actives.

9. Produit selon la revendication 8, dans lequel le minéral osseux contient de la taurolidine et/ou du taurultame.

10. Produit selon la revendication 8, dans lequel le minéral osseux ou les fibres de collagène contiennent une ou plusieurs protéines ou un ou plusieurs polypeptides capables d'aider à la régénération osseuse.

11. Produit selon la revendication 10, dans lequel les protéines ou les polypeptides englobent des facteurs de croissance mitogènes, morphogènes ou angiogènes, un facteur de croissance osseuse en transformation, l'osséine ou l'ostéopoïétine.

12. Produit selon l'une quelconque des revendications précédentes, qui est sous forme de particules de diamètre moyen dans la gamme de 0,1 à 10 mm.
